# EUROPÄISCHE PATENTSCHRIFT

(11) **EP 0 525 351 B1**
(45) Veröffentlichungstag und Bekanntmachung des Hinweises auf die Patenterteilung: **05.04.1995**
(21) Anmeldenummer: 92110017.8
(22) Anmeldetag: 13.06.1992
(51) Int. Cl.: C07C 69/734, C07C 67/31, C25B 3/02

(54) **2-Aryl-2,2-dialkoxyessigsäurealkylester, Verfahren zu ihrer Herstellung und ihre Verwendung zur Herstellung von Arylglyoxylsäureestern**
2-Aryle-2,2-dialkoxyacetic acid alkyle esters, process for their preparation and use in the preparation of esters of aryle glyoxylic acid
Esters alkylique d'acide 2-aryle-2,2-dialcoxyacétique, procédé pour leur préparation et utilisation dans la préparation d'esters d'acide aryleglyoxylique

(30) Priorität: 05.07.1991 DE 4122315
(43) Veröffentlichungstag der Anmeldung: 03.02.1993
(73) Patentinhaber: BASF Aktiengesellschaft, 67063 Ludwigshafen (DE)
(72) Erfinder: Hermeling, Dieter, Dr., W-6710 Frankenthal (DE)

(56) Entgegenhaltungen:
- EP-A- 0 287 954
- LIEBIGS ANNALEN DER CHEMIE Nr. 5, 1980, WEINHEIM,DE Seiten 1271 - 1282 S ANTUS ET AL 'Oxidation von 1,3-Diphenyl-1,3-propandionen mit Thallium-(III)-nitrat in Methanol'
- LIEBIGS ANNALEN DER CHEMIE Nr. 2, 1990, WEINHEIM,DE Seiten 181 - 183 J KAWABATA ET AL 'Electrochemical hydroxylation,methoxylation and hydroxymethylation of active methine compounds'
- CHEMICAL AND PHARMACEUTICAL BULLETIN Bd. 40, Nr. 4, April 1992, TOKYO,JP Seiten 1037 - 1039 M KATO ET AL 'Electrochemical methoxylation of arylacrylates'

## Beschreibung

Die Erfindung betrifft 2-Aryl-2,2-dialkoxyessigsäurealkylester, ein Verfahren zu ihrer Herstellung durch elektrochemische Oxidation und ihre Verwendung zur Herstellung von Arylglyoxylsäureestern.

Dialkylacetale von Arylglyoxylsäureestern lassen sich z.B. nach Synthesis 1983, 203 - 205 durch Umsetzung von Arylglyoxylsäureestern mit Methanol in Gegenwart von Chlortrimethylsilan oder nach Tetrahedron Lett. 21, 4997 (1980) durch eine kombinierte säurekatalysierte Acetalisierung-Veresterung von Arylglyoxylsäuren herstellen.

Da aber nicht die Acetale, sondern die Arylglyoxylsäureester selbst die interessanteren Verbindungen sind, die beschriebenen Acetalisierungen aber von den Arylglyoxylsäuren bzw. -estern ausgehen, besitzen diese Synthesen keine wirtschaftliche Bedeutung.

Der 2,2-Dimethoxy-2-(4-methoxyphenyl)essigsäuremethylester entsteht als Abbauprodukt bei der Oxidation eines 1,3-Diphenyl-1,3-propandions mit Thallium(III)-nitrat (Liebigs Ann. Chem. 1980, 1271-1282). Diese Reaktion hat aber aufgrund des speziellen Oxidationsmittels keine wirtschaftliche Bedeutung.

Die Acetale stellen jedoch, sofern sie einfach synthetisiert werden können, wichtige Vorstufen für die Arylglyoxylsäureester dar, da sie einerseits die Ketogruppe vor einer ungewollten Reaktion schützen, gleichzeitig aber Umsetzungen an der Estergruppierung erlauben, und da sie andererseits sehr leicht und in nahezu quantitativer Ausbeute zu den entsprechenden Arylglyoxylsäureestern umgewandelt werden können. Die Arylglyoxylsäureester sind Verbindungen mit vielfältigen Verwendungsmöglichkeiten, die z.B. nach EP 242 081 als Vorprodukte für Herbizide, Fungizide, Insektizide, nach EP 25 271 als Vorprodukte für Antibiotika oder nach JR 61 097 247 als Vorprodukte für α-Aminosäuren dienen.

Der Erfindung lag daher die Aufgabe zugrunde, Arylglyoxylsäurealkylesterdialkylacetale einfach zugänglich zu machen.

Es wurde nun gefunden, daß sich 2-Aryl-2,2-dialkoxyessigsäurealkylester der allgemeinen Formel I 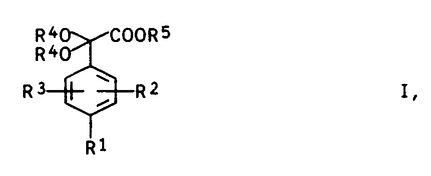
in der R¹ einen gerad- oder verzweigtkettigen C₁-C₁₂-Alkyl-, C₃-C₁₂-Cycloalkyl-, C₄-C₁₂-Alkyl-Cycloalkyl-, C₁-C₁₂-Alkoxy- oder C₆-C₁₄-Aryloxy-Rest; R², R³ unabhängig voneinander Wasserstoff, Halogen oder einen gerad- oder verzweigtkettigen C₁-C₁₂-Alkyl-, C₁-C₁₂-Alkoxy-, C₃-C₁₂-Cycloalkyl-, C₄-C₁₂-Alkyl-Cycloalkyl-, C₆-C₁₄-Aryloxy-, C₆-C₁₄-Aryl-Rest; R⁴ einen C₁-C₆-Alkyl-Rest und R⁵ einen gerad- oder verzweigtkettigen C₁-C₂₀-Alkyl-Rest bedeuten, einfach herstellen lassen, indem man Arylessigsäurealkylester der allgemeinen Formel II 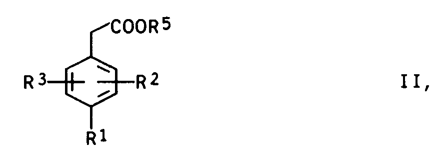
in der R¹, R², R³, R⁵ die oben genannten Bedeutungen haben, elektrochemisch in Gegenwart eines Alkohols R⁴OH, wobei R⁴ für einen C₁-C₆-Alkyl-Rest steht, oxidiert.

Die auf diese Weise hergestellten 2-Aryl-2,2-dialkoxyessigsäurealkylester der Formel I sind neu, wobei jedoch R¹ nicht Methoxy sein kann, wenn R² und R³ für Wasserstoff und R⁴ und R⁵ für Methyl stehen.

Für die Substituenten R¹ bis R⁵ kommen folgende Gruppierungen in Betracht:
- R1: gerad- oder verzweigtkettiges C₁-C₁₂-Alkyl, vorzugsweise C₁-C₈-Alkyl wie Methyl, Ethyl, n-Propyl, iso-Propyl, n-Butyl, iso-Butyl, sek.-Butyl, tert.-Butyl, n-Pentyl, tert.-Amyl, 1,1-Dimethylbutyl, 1,1-Dimethylpentyl, 1,1-Dimethylhexyl, 1,1,2,2-Tetramethylpropyl, besonders bevorzugt tert.-Butyl, tert.-Amyl, 1,1,2,2-Tetramethylpropyl,
gerad- oder verzweigtkettiges C₁-C₁₂-Alkoxy, vorzugsweise C₁-C₈-Alkoxy wie Methoxy, Ethoxy, Propyloxy, n-Butyloxy, iso-Butyloxy, sek.-Butyloxy, tert.-Butyloxy, n-Pentyloxy, tert.-Amyloxy, n-Hexyloxy, n-Heptyloxy, n-Octyloxy, besonders bevorzugt Methoxy, Ethoxy, tert.-Butyloxy, n-Hexyloxy,
Cycloalkylreste mit 3 bis 12 C-Atomen, bevorzugt Cyclopentyl, Cyclohexyl, Cycloheptyl, oder
Alkyl-Cycloalkylreste mit 4 bis 12 C-Atomen, wie 1-Methylcyclopent-1-yl, 1-Methylcyclohex-1-yl, 1-Ethylcyclohex-1-yl, 1,4-Dimethylcyclohex-1-yl,
1,3-Dimethylcyclohex-1-yl, 1,3,5-Trimethylcyclohex-1-yl, 1-Isopropylcyclohex-1-yl, 1-tert.-Butylcyclohex-1-yl, besonders bevorzugt 1-Methylcyclopent-1-yl,
1-Methyl-cyclohex-1-yl, 1,3,5-Trimethylcyclohex-1-yl,
C₆-C₁₄-Aryloxy wie Phenoxy, 1-Naphthyloxy, 2-Naphthyloxy, besonders bevorzugt Phenoxy,
- R², R³: unabhängig voneinander Wasserstoff,
gerad- oder verzweigtkettiges C₁-C₁₂-Alkyl, vorzugsweise C₁-C₈-Alkyl wie Methyl, Ethyl, n-Propyl, iso-Propyl, n-Butyl, iso-Butyl, sek.-Butyl, tert.-Butyl, n-Pentyl, tert.-Amyl, 1,1-Dimethylbutyl, 1,1-Dimethylpentyl, 1,1-Dimethylhexyl, 1,1,2,2-Tetramethylpropyl, besonders bevorzugt tert.-Butyl, tert.-Amyl, 1,1,2,2-Tetramethylpropyl,
gerad- oder verzweigtkettiges C₁-C₁₂-Alkoxy, vorzugsweise C₁-C₈-Alkoxy wie Methoxy, Ethoxy, Propyloxy, n-Butyloxy, iso-Butyloxy, sek.-Butyloxy, tert.-Butyloxy, n-Pentyloxy, tert.-Amyloxy, n-Hexyloxy, n-Heptyloxy, n-Octyloxy, besonders bevorzugt Methoxy, Ethoxy, tert.-Butyloxy, n-Hexyloxy,
C₃-C₁₂-Cycloalkyl, vorzugsweise C₃-C₇-Cycloalkyl wie Cyclopropyl, Cyclobutyl, Cyclopentyl, Cyclohexyl, Cycloheptyl, besonders bevorzugt Cyclopentyl, Cyclohexyl,
Alkyl-Cycloalkylreste mit 4 bis 12 C-Atomen, wie 1-Methylcyclopent-1-yl, 1-Methylcyclohex-1-yl, 1-Ethylcyclohex-1-yl, 1,4-Dimethylcyclohex-1-yl, 1,3-Dimethylcyclohex-1-yl, 1,3,5-Trimethylcyclohex-1-yl, 1-Isopropylcyclohex-1-yl, 1-tert.-Butylcyclohex-1-yl, besonders bevorzugt 1-Methylcyclopent-1-yl, 1-Methyl-cyclohex-1-yl, 1,3,5-Trimethylcyclohex-1-yl,
C₆-C₁₄-Aryloxy wie Phenoxy, 1-Naphthyloxy, 2-Naphthyloxy, bevorzugt Phenoxy,
C₆-C₁₄-Aryl wie Phenyl, 1-Naphthyl, 2-Naphthyl, bevorzugt Phenyl,
Halogen wie Fluor, Chlor, Brom, bevorzugt Fluor und Chlor,
- R⁴: geradkettiges C₁-C₆-Alkyl, vorzugsweise C₁-C₄-Alkyl wie Methyl, Ethyl, Propyl, Butyl, besonders bevorzugt Methyl und Ethyl,
- R⁵: gerad- oder verzweigtkettiges C₁-C₂₀-Alkyl, vorzugweise C₁-C₁₂-Alkyl wie Methyl, Ethyl, n-Propyl, iso-Propyl, n-Butyl, iso-Butyl, sek.-Butyl, tert.-Butyl, n-Pentyl, tert.-Amyl, n-Hexyl, n-Heptyl, n-Octyl, n-Nonyl, n-Decyl, n-Undecyl, n-Dodecyl, besonders bevorzugt Methyl, Ethyl, iso-Propyl, tert.-Butyl.

Für das beanspruchte Verfahren lassen sich die Substituenten beliebig kombinieren, für die Substituenten R¹, R² und R³ ist es jedoch bevorzugt, daß die α-Atome kein Wasserstoff tragen, da durch die Wasserstoffe an den α-C-Atomen die Ausbeuten der elektrochemischen Oxidation aufgrund des Auftretens von Nebenprodukten sinken.

Bevorzugte Verbindungen sind:
2,2-Dimethoxy-2-(4-methoxyphenyl)essigsäureethylester
2,2-Dimethoxy-2-(4-methoxyphenyl)essigsäure-tert.-butylester
2,2-Diethoxy-2-(4-methoxyphenyl)essigsäuremethylester
2,2-Diethoxy-2-(4-methoxyphenyl)essigsäure-tert.-butylester
2,2-Dimethoxy-2-(4-ethoxyphenyl)essigsäuremethylester
2,2-Dimethoxy-2-(4-ethoxyphenyl)essigsäureethylester
2,2-Dimethoxy-2-(4-ethoxyphenyl)essigsäure-tert.-butylester
2,2-Diethoxy-2-(4-ethoxyphenyl)essigsäuremethylester
2,2-Diethoxy-2-(4-ethoxyphenyl)essigsäureethylester
2,2-Diethoxy-2-(4-ethoxyphenyl)essigsäure-tert.-butylester
2,2-Dimethoxy-2-(4-hexyloxyphenyl)essigsäuremethylester
2,2-Dimethoxy-2-(4-hexyloxyphenyl)essigsäure-tert.-butylester
2,2-Dimethoxy-2-(4-hexyloxyphenyl)essigsäureethylester
2,2-Diethoxy-2-(4-hexyloxyphenyl)essigsäuremethylester
2,2-Diethoxy-2-(4-hexyloxyphenyl)essigsäureethylester
2,2-Diethoxy-2-(4-hexyloxyphenyl)essigsäure-tert.-butylester
2,2-Dimethoxy-2-(4-tert.-butyloxyphenyl)essigsäuremethylester
2,2-Dimethoxy-2-(4-tert.-butyloxyphenyl)essigsäureethylester
2,2-Dimethoxy-2-(4-tert.-butyloxyphenyl)essigsäure-tert.-butylester
2,2-Diethoxy-2-(4-tert.-butyloxyphenyl)essigsäuremethylester
2,2-Diethoxy-2-(4-tert.-butyloxyphenyl)essigsäureethylester
2,2-Diethoxy-2-(4-tert.-butyloxyphenyl)essigsäure-tert.-butylester
2,2-Dimethoxy-2-(4-phenoxyphenyl)essigsäuremethylester
2,2-Dimethoxy-2-(4-phenoxyphenyl)essigsäure-tert.-butylester
2,2-Dimethoxy-2-(4-phenoxyphenyl)essigsäureethylester
2,2-Diethoxy-2-(4-phenoxyphenyl)essigsäuremethylester
2,2-Diethoxy-2-(4-phenoxyphenyl)essigsäureethylester
2,2-Diethoxy-2-(4-phenoxyphenyl)essigsäure-tert.-butylester
2,2-Dimethoxy-2-(3,4-dimethoxyphenyl)essigsäuremethylester
2,2-Dimethoxy-2-(3,4-dimethoxyphenyl)essigsäureethylester
2,2-Dimethoxy-2-(3,4-dimethoxyphenyl)essigsäure-tert.-butylester
2,2-Diethoxy-2-(3,4-dimethoxyphenyl)essigsäuremethylester
2,2-Diethoxy-2-(3,4-dimethoxyphenyl)essigsäureethylester
2,2-Diethoxy-2-(3,4-dimethoxyphenyl)essigsäure-tert.-butylester
2,2-Dimethoxy-2-(4-tert.-butylphenyl)essigsäuremethylester
2,2-Dimethoxy-2-(4-tert.-butylphenyl)essigsäureethylester
2,2-Dimethoxy-2-(4-tert.-butylphenyl)essigsäure-tert.-butylester
2,2-Diethoxy-2-(4-tert.-butylphenyl)essigsäuremethylester
2,2-Diethoxy-2-(4-tert.-butylphenyl)essigsäureethylester
2,2-Diethoxy-2-(4-tert.-butylphenyl)essigsäure-tert.-butylester
Die elektrochemischen Oxidationen können in den technisch üblichen Elektrolysezellen durchgeführt werden. Bevorzugt werden ungeteilte Durchflußzellen verwendet. Als Anoden kommen z. B. Edelmetallelektroden wie Platin oder Oxidelektroden wie Ti/RuOₓ, RuO₂ oder Cr₂O₃ in Frage. Bevorzugtes Anodenmaterial ist Graphit. Als Kathoden kommen z.B. Stahl, Eisen, Kupfer, Nickel, Zink und Kohle sowie Edelmetalle wie Platin in Betracht. Bevorzugtes Kathodenmaterial ist Graphit. Der Elektrolyt setzt sich aus der Ausgangsverbindung der Formel II, dem Alkohol R⁴OH und einem Hilfselektrolyten zusammen. Als Hilfselektrolyte kommen Neutralsalze, Säuren und Basen in Betracht. Beispiele für Neutralsalze sind Fluoride wie KF, Sulfonate wie NaSO₃Ph, Sulfate wie (CH₃)₄NSO₄CH₃, Tetrafluoroborate wie NaBF₄, Phosphate und Phosphonate. Beispiele für Säuren sind Schwefelsäure, Alkyl- und Arylsulfonsäuren wie Methyl- oder Benzolsulfonsäure. Als Basen dienen z. B. Alkoholate wie NaOCH₃ oder Hydroxide wie KOH.

Der Elektrolyt hat beispielsweise folgende Zusammensetzung:
1 bis 49, vorzugsweise 5 bis 30 Gew.-% Arylessigsäurealkylester der Formel II,
0,1 bis 5, vorzugsweise 0,2 bis 3 Gew.-% Hilfselektrolyt
50 bis 98,9, vorzugsweise 70 bis 95 Gew.-% Alkohol R⁴OH
Die Stromdichte kann bei dem erfindungsgemäßen Verfahren in weiten Grenzen, z.B. von 0,1 bis 25 A/dm², bevorzugt von 1 bis 10 A/dm² gewählt werden. Auch die Temperaturen können in weiten Grenzen variiert werden. So können die Oxidationen bei 0 bis 120°C, vorzugsweise bei 20 bis 80°C durchgeführt werden. Die Elektrolysetemperatur ist u.a. vom Alkohol R⁴OH abhängig. Es wird in jedem Fall bei Temperaturen unterhalb des Siedepunktes des Alkohols R⁴OH gearbeitet. Die Elektrolysen werden bevorzugt bei Normaldruck durchgeführt, es kann aber auch unter Druck bis zu 10 bar elektrolysiert werden. Durch die damit verbundene Siedepunktserhöhung kann z.B. auch in Methanol bei Temperaturen oberhalb von 60°C elektrolysiert werden.

Die 2-Arylessigsäurealkylester der Formel II sind z.T. käuflich erhältlich oder können in an sich üblicher Weise aus den entsprechend substituierten Essigsäuren hergestellt werden.

Die 2-Arylessigsäurealkylester der Formel II lassen sich weitestgehend umsetzen. Die Elektrolysen können sowohl kontinuierlich als auch diskontinuierlich durchgeführt werden. Auftretende Zwischenprodukte (Ether) sowie nicht umgesetzte Edukte können in die Reaktion zurückgeführt werden. Die Aufarbeitung der Elektrolyseausträge erfolgt nach konventionellen Methoden, vorzugsweise wird destillativ aufgearbeitet.

Aus den erhaltenen 2-Aryl-2,2-dialkoxyessigsäurealkylestern lassen sich durch Hydrolyse in an sich bekannter Weise die Arylglyoxylsäureester herstellen (s. z.B. Houben Weyl, Methoden der organischen Chemie, 4. Aufl. Georg-Thieme-Verlag, Stuttgart, 1954, Sauerstoffverbindungen II, 7/1, Aldehyde, S. 423-428).

### Beispiele

### Beispiel 1

### Elektrosynthese von 2,2-Dimethoxy-2-(4-methoxyphenyl)essigsäuremethylester

- Apparatur:: ungeteilte Zelle mit 11 bipolaren Elektroden
- Anoden:: Graphit
- Elektrolyt:: 300 g (1,667 mol) 2-(4-Methoxyphenyl)essigsäure methylester, 30 g Natriumbenzolsulfonat und 2670 g Methanol
- Kathoden:: Graphit
- Elektrolysetemperatur:: 40°C

Elektrolyse mit 5,6 F/mol 2-(4-Methoxyphenyl)essigsäuremethylester.

Der Elektrolyt wird während der Elektrolyse mit 200 l/h durch die Zelle gepumpt.

### Aufarbeitung:

Nach Beendigung der Elektrolyse wird das Methanol bei Normaldruck abdestilliert, das Leitsalz abfiltriert und das Filtrat im Vakuum reindestilliert. Man erhält 9,8 g (47 mmol; 3 %) 2-Methoxy-2-(4-methoxyphenyl)essigsäuremethylester und 240,2 g (1,001 mol; 60 %) 2,2-Dimethoxy-2-(4-methoxyphenyl)essigsäuremethylester. Der 2-Methoxy-2-(4-methoxyphenyl)essigsäuremethylester wird als Zwischenprodukt in die Reaktion zurückgeführt. Hieraus errechnet sich dann eine Selektivität von 62 %.
Sdp.: 123°C/8 mbar

### Beispiel 2

### Elektrosynthese von 2,2-Dimethoxy-2-(3,4-dimethoxyphenyl)essigsäuremethylester

2-(3,4-Dimethoxyphenyl)essigsäuremethylester wird wie in Beispiel 1 beschrieben elektrolysiert und aufgearbeitet.
- Elektrolyt:: 300 g (1,429 mol) 2-(3,4-Dimethoxyphenyl) essigsäuremethylester, 30 g Natriumbenzolsulfonat und 2670 g Methanol
- Elektrolysetemperatur:: 52°C
Elektrolyse mit 6 F/mol 2-(3,4-Dimethoxyphenyl)essigsäuremethylester
Der Elektrolyt wird während der Elektrolyse mit 200 l/h durch die Zelle gepumpt.

Nach Aufarbeitung und Reindestillation im Vakuum erhält man 280,6 g (1,039 mol; 73 %) 2,2-Dimethoxy-2-(3,4-dimethoxyphenyl)essigsäuremethylester.
Sdp.: 153°C/1 mbar

### Beispiel 3

### Elektrosynthese von 2,2-Diethoxy-2-(4-n-hexyloxyphenyl)essigsäureethylester

2-(4-n-Hexyloxyphenyl)essigsäureethylester wird wie in Beispiel 1 beschrieben elektrolysiert und aufgearbeitet.
- Anzahl der bipolaren Elektroden:: 9
- Elektrolyt:: 93,8 g (355 mmol) 2-(4-n-Hexyloxyphenyl)essigsäureethylester, 2,1 g Natriumbenzolsulfonat und 529 g Ethanol
- Elektrolysetemperatur:: 50°C
Elektrolyse mit 8 F/mol 2-(4-n-Hexyloxyphenyl)essigsäureethylester.

Der Elektrolyt wird während der Elektrolyse mit 20 l/h durch die Zelle gepumpt.

Nach Aufarbeitung und Reindestillation im Vakuum erhält man 6,6 g (21 mmol; 6 %) 2-Ethoxy-2-(4-n-hexyloxyphenyl)essigsäureethylester (wird in die Reaktion zurückgeführt) und 66,2 g (188 mmol; 53 %) 2,2-Diethoxy-2-(4-n-hexyloxyphenyl)essigsäureethylester. Hieraus errechnet sich eine Selektivität von 56 %.
Sdp.: 168°C/1 mbar

### Beispiel 4

### Elektrosynthese von 2,2-Diethoxy-2-(4-methoxyphenyl)essigsäureethylester

2-(4-Methoxyphenyl)essigsäureethylester wird wie in Beispiel 1 beschrieben elektrolysiert und aufgearbeitet.
- Anzahl der bipolaren Elektroden:: 9
- Elektrolyt:: 93,8 g (484 mmol) 2-(4-Methoxyphenyl)essigsäureethylester, 2 g Natriumbenzolsulfonat und 525 g Ethanol
- Elektrolysetemperatur:: 48°C
Elektrolyse mit 7 F/mol 2-(4-Methoxyphenyl)essigsäureethylester.

Der Elektrolyt wird während der Elektrolyse mit 20 l/h durch die Zelle gepumpt.

Nach Aufarbeitung und Reindestillation im Vakuum erhält man 7,6 g (32 mmol; 7 %) 2-Ethoxy-2-(4-methoxyphenyl)essigsäureethylester (wird in die Reaktion zurückgeführt) und 86,8 g (308 mmol; 64 %) 2, 2-Diethoxy-2-(4-methoxyphenyl)essigsäureethylester. Hieraus errechnet sich eine Selektivität von 69 %.
Sdp.: 143°C/3 mbar

### Beispiel 5

### Elektrosynthese von 2,2-Dimethoxy-2-(4-methoxyphenyl)essigsäureethylester

2-(4-Methoxyphenyl)essigsäureethylester wird wie in Beispiel 1 beschrieben elektrolysiert und aufgearbeitet.
- Anzahl der bipolaren Elektroden:: 9
- Elektrolyt:: 93,8 g (484 mmol) 2-(4-Methoxyphenyl)essigsäureethylester, 6,2 g Natriumbenzolsulfonat und 525 g Methanol
- Elektrolysetemperatur:: 14°C
Elektrolyse mit 6 F/mol 2-(4-Methoxyphenyl)essigsäureethylester.

Der Elektrolyt wird während der Elektrolyse mit 20 l/h durch die Zelle gepumpt.

Nach Aufarbeitung und Reindestillation im Vakuum erhält man 7,9 g (35 mmol; 7 %) 2-Methoxy-2-(4-methoxyphenyl)essigsäureethylester (wird in die Reaktion zurückgeführt) und 74,5 g (293 mmol; 61 %) 2,2-Dimethoxy-2-(4-methoxyphenyl)essigsäureethylester. Hieraus errechnet sich eine Selektivität von 66 %.
Sdp.: 131°C/3 mbar

### Beispiel 6

### Elektrosynthese von 2,2-Dimethoxy-2-(4-tert.-butylphenyl)essigsäuremethylester

2-(4-tert.-Butylphenyl)essigsäuremethylester wird wie in Beispiel 1 beschrieben elektrolysiert und aufgearbeitet.
- Anzahl der bipolaren Elektroden:: 9
- Elektrolyt:: 93,8 g (455 mmol) 2-(4-tert.-Butylphenyl)essigsäuremethylester, 6,2 g Natriumbenzolsulfonat und 525 g Methanol.
- Elektrolysetemperatur:: 48°C
Elektrolyse mit 34 F/mol 2-(4-tert.-Butylphenyl)essigsäuremethylester
Der Elektrolyt wird während der Elektrolyse mit 20 l/h durch die Zelle gepumpt.

Nach Aufarbeitung und Reindestillation im Vakuum erhält man 6,0 g (25 mmol; 6 %) 2-Methoxy-2-(4-tert.-butylphenyl)essigsäuremethylester (wird in die Reaktion zurückgeführt) und 45,8 g (172 mmol; 38 %) 2,2-Dimethoxy-2-(4-tert.-butylphenyl)essigsäuremethylester. Hieraus errechnet sich eine Selektivität von 40 %.
Sdp.: 142°C/3 mbar

## Patentansprüche

1. 2-Aryl-2,2-dialkoxyessigsäurealkylester der allgemeinen Formel I 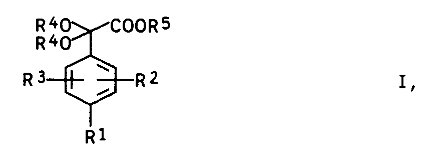 in der
R¹ einen gerad- oder verzweigtkettigen C₁-C₁₂-Alkyl-, C₃-C₁₂-Cycloalkyl-, C₄-C₁₂-Alkyl-Cycloalkyl-, C₁-C₁₂-Alkoxy- oder C₆-C₁₄-Aryloxy-Rest;
R², R³ unabhängig voneinander Wasserstoff, Halogen oder einen gerad- oder verzweigtkettigen C₁-C₁₂-Alkyl-, C₁-C₁₂-Alkoxy-, C₃-C₁₂-Cycloalkyl-, C₄-C₁₂-Alkyl-Cycloalkyl-, C₆-C₁₄-Aryloxy- oder C₆-C₁₄-Aryl-Rest;
R⁴ einen C₁-C₆-Alkyl-Rest und
R⁵ einen gerad- oder verzweigtkettigen C₁-C₂₀-Alkyl-Rest bedeuten,
mit der Maßgabe, daß R¹ nicht Methoxy sein kann, wenn R² und R³ für Wasserstoff und R⁴ und R⁵ für Methyl stehen.

2. 2-Aryl-2,2-dialkoxyessigsäurealkylester gemäß Anspruch 1, dadurch gekennzeichnet, daß der Alkylrest R¹ und die Alkyl- sowie Cycloalkylreste R² und R³ C₄-C₁₂-Reste ohne einen Wasserstoff am α-C-Atom sind.

3. 2-Aryl-2,2-dialkoxyessigsäurealkylester gemäß Anspruch 1, dadurch gekennzeichnet, daß der Rest R¹ ein C₁-C₆-Alkoxy-Rest ist und/oder die Reste R⁴ und R⁵ unabhängig voneinander einen Methyl- oder Ethyl-Rest bedeuten.

4. Verfahren zur Herstellung von 2-Aryl-2,2-dialkoxyessigsäurealkylestern der allgemeinen Formel I, in der R¹, R², R³, R⁵ die in den Ansprüchen 1 bis 3 genannten Bedeutungen haben, wobei die Reste R¹, R², R³, R⁴, R⁵ zusätzlich die durch die Maßgabe in Anspruch 1 ausgeschlossene Bedeutung haben können, dadurch gekennzeichnet, daß man 2-Arylessigsäurealkylester der allgemeinen Formel II 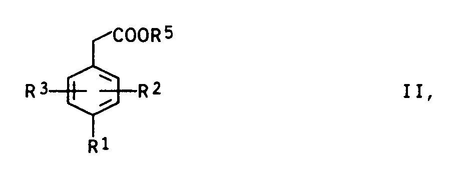 in der R¹, R², R³ und R⁵ die oben genannten Bedeutungen haben, elektrochemisch in Gegenwart eines Alkohols R⁴OH, wobei R⁴ für einen C₁-C₆-Alkyl-Rest steht, oxidiert.

5. Verfahren nach Anspruch 4, dadurch gekennzeichnet, daß man die Elektrolysen in ungeteilten Zellen durchführt.

6. Verfahren nach Anspruch 4, dadurch gekennzeichnet, daß man die Elektrolysen an Graphitanoden durchführt.

7. Verfahren nach Anspruch 4, dadurch gekennzeichnet, daß man die Elektrolysen bei Temperaturen von 0 bis 120°C durchführt.

8. Verfahren nach Anspruch 4, dadurch gekennzeichnet, daß man die Elektrolysen bei Normaldruck oder bei einem Überdruck bis zu 10 bar durchführt.

9. Verfahren nach Anspruch 4, dadurch gekennzeichnet, daß man die Elektrolysen bei Stromdichten von 0,1 bis 25 A/dm² durchführt.

10. Verwendung der 2-Aryl-2,2-dialkoxyessigsäurealkylestern, hergestellt nach dem Verfahren gemäß Anspruch 4, zur Herstellung von Arylglyoxylsäureestern.

## Claims

1. An alkyl 2-aryl-2,2-dialkoxyacetate of the formula I 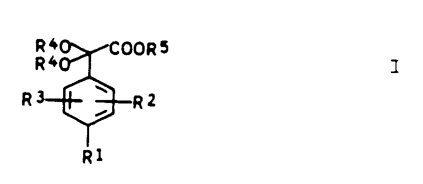 where
R¹ is straight-chain or branched C₁-C₁₂-alkyl, C₃-C₁₂-cycloalkyl, C₄-C₁₂-alkyl-cycloalkyl, C₁-C₁₂-alkoxy or C₆-C₁₄-aryloxy;
R² and R³ are, independently of one another, hydrogen, halogen or straight-chain or branched C₁-C₁₂-alkyl, C₁-C₁₂-alkoxy, C₃-C₁₂-cycloalkyl, C₄-C₁₂-alkyl-cycloalkyl, C₆-C₁₄-aryloxy or C₆-C₁₄-aryl;
R⁴ is C₁-C₆-alkyl, and
R⁵ is straight-chain or branched C₁-C₂₀-alkyl,
with the proviso that R¹ cannot be methoxy when R² and R³ are hydrogen and R⁴ and R⁵ are methyl.

2. An alkyl 2-aryl-2,2-dialkoxyacetate as claimed in claim 1, where the alkyl radical R¹ and the alkyl and cycloalkyl radicals R² and R³ are C₄-C₁₂ radicals without a hydrogen on the α-C atom.

3. An alkyl 2-aryl-2,2-dialkoxyacetate as claimed in claim 1, where R¹ is C₁-C₆-alkoxy and/or R⁴ and R⁵ are, independently of one another, methyl or ethyl.

4. A process for preparing alkyl 2-aryl-2,2-dialkoxyacetates of the formula I where R¹, R², R³ and R⁵ have the meanings specified in claim 1 or 2 or 3 and where R¹, R², R³, R⁴ and R⁵ can additionally have the meanings excluded by the proviso in claim 1, which comprises oxidizing alkyl 2-arylacetates of the formula II 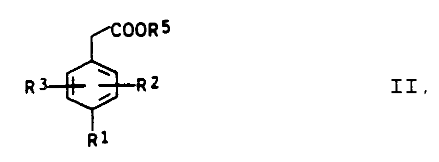 where R¹, R², R³ and R⁵ have the abovementioned meanings, electrochemically in the presence of an alcohol R⁴OH, where R⁴ is C₁-C₆-alkyl.

5. A process as claimed in claim 4, wherein the electrolyses are carried out in undivided cells.

6. A process as claimed in claim 4, wherein the electrolyses are carried out at graphite anodes.

7. A process as claimed in claim 4, wherein the electrolyses are carried out at from 0 to 120°C.

8. A process as claimed in claim 4, wherein the electrolyses are carried out under atmospheric pressure or superatmospheric pressure up to 10 bar.

9. A process as claimed in claim 4, wherein the electrolyses are carried out with current densities of from 0.1 to 25 A/dm².

10. A method of using the alkyl 2-aryl-2,2-dialkoxyacetates prepared by the process as claimed in claim 4 for the preparation of arylglyoxylic esters by hydrolysis.

## Revendications

1. Esters alkyliques d'acide 2-aryl-2,2-dialcoxyacétique de formule générale I 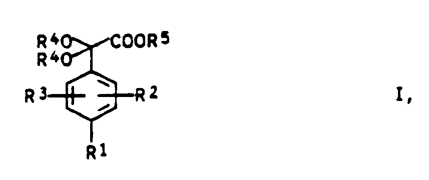 dans laquelle
R¹ représente un reste alkyle en C₁-C₁₂ à chaîne droite ou ramifiée, cycloalkyle en C₃-C₁₂, alkylcycloalkyle en C₄-C₁₂, alcoxy en C₁-C₁₂ ou aryloxy en C₆-C₁₄;
R², R³ représentent chacun, indépendamment l'un de l'autre, un atome d'hydrogéné d'halogène ou un reste alkyle en C₁-C₁₂ à chaîne droite ou ramifiée, alcoxy en C₁-C₁₂, cycloalkyle en C₃-C₁₂, alkylcycloalkyle en C₄-C₁₂, aryloxy en C₆-C₁₄ ou aryle en C₆-C₁₄;
R⁴ représente un reste alkyle en C₁-C₆; et
R⁵ réprésente un reste alkyle en C₁-C₂₀ a chaîne droite ou ramifiée,
étant spécifié que R¹ ne peut pas être un reste méthoxy lorsque R² et R³ sont mis chacun pour un atome d'hydrogène et que R⁴ et R⁵ sont mis chacun pour un reste méthyle.

2. Esters alkyliques d'acide 2-aryl-2,2-dialcoxyacétique selon la revendication 1, caractérisés en ce que le reste alkyle R¹ et les restes alkyle et cycloalkyle R² et R³ sont des restes en C₄-C₁₂ sans atome d'hydrogène sur l'atome de carbone en position α.

3. Esters alkyliques d'acide 2-aryl-2,2-dialcoxyacétique selon la revendication 1, caractérisés en ce que le reste R¹ est un reste alcoxy en C₁-C₆ et/ou les restes R⁴ et R⁵ représentent chacun, indépendamment l'un de l'autre, un reste méthyle ou éthyle.

4. Procedé de préparation d'esters alkyliques d'acide 2-aryl-2,2-dialcoxyacétique de formule générale I, dans laquelle R¹, R², R³, R⁵ ont les significations données dans les revendications 1 à 3, les restes R¹, R², R³, R⁴, R⁵ pouvant en plus avoir la signification exclue par la condition spécifiée dans la revendication 1, caractérisé en ce qu'on oxyde par voie électrochimique des esters alkyliques d'acide 2-arylacetique de formule générale II 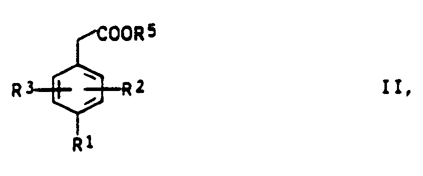 dans laquelle R¹, R², R³ et R⁵ ont les significations données ci-dessus, en présence d'un alcool R⁴OH dans lequel R⁴ est mis tour un reste alkyle en C₁-C₆.

5. Procédé selon la revendication 4, caractérisé en ce qu'on effectue l'électrolyse dans des cellules non divisées.

6. Procédé selon la revendication 4, caractérisé en ce qu'on effectue l'électrolyse sur des anodes en graphite.

7. Procédé selon la revendication 4, caractérisé en ce qu'on conduit l'électrolyse à des températures de 0 à 120°C.

8. Procédé selon la revendication 4, caractérisé en ce qu'on conduit l'électrolyse sous la pression normale ou sous une surpression pouvant aller jusqu'à 10 bar.

9. Procédé selon la revendication 4, caractérisé en ce qu'on conduit l'électrolyse avec des densités de courant de 0>1 à 25 A/dm².

10. Utilisation des esters alkyliques d'acide 2-aryl-2,2-dialcoxyacétique, prépares par le procédé selon la revendication 4, pour la préparation d'esters d'acide arylglyoxylique.
